# EUROPEAN PATENT APPLICATION

(11) **EP 3 235 439 A1**
(43) Date of publication of application: **25.10.2017**
(21) Application number: 15869640.1
(22) Date of filing: 13.10.2015
(51) Int. Cl.: A61B 8/14, G06Q 50/24

(54) **ULTRASONIC DIAGNOSTIC DEVICE AND METHOD FOR PRODUCING EXAMINATION REPORT**

(30) Priority: 17.12.2014 JP 2014255293
(71) Applicant: Hitachi, Ltd., Chiyoda-ku, Tokyo 100-8280 (JP)
(72) Inventor: TAJIMA, Fumie, Mitaka-shi Tokyo 181-8622 (JP); MITA, Yukihiro, Mitaka-shi Tokyo 181-8622 (JP)
(74) Representative: Strehl Schübel-Hopf & Partner
(86) International application number: PCT/JP2015/078937
(87) International publication number: WO 2016/098432

(57) **Abstract**

According to the present invention, the production of repeated examination reports for each examination is limited in a report producing unit. In an ultrasonic diagnostic device according to the present invention, when a user provides an instruction to start producing a report, the report producing unit is activated. For example, the user inputs observations and the like into an examination report. When there is an instruction to finish producing the report from the user, the report producing unit produces an examination report indicating content at the time the instruction is received. When the examination is finished, the examination report is automatically transmitted to a work station. When an examination report is not produced at the time the examination is finished, the report producing unit automatically produces the examination report. In this case, the examination report is also automatically transmitted to the work station.

## Description

### TECHNICAL FIELD

The present invention relates to an ultrasonic diagnostic device, and in particular to a technique for producing and managing an examination report.

### BACKGROUND

In general, when an examination is conducted using an ultrasonic diagnostic device, an examination report reflecting results of the examination is produced. For example, ultrasound image data and measurement data obtained through the examination are transmitted from the ultrasonic diagnostic device to a workstation. In the workstation, an examination report is produced by referencing the transmitted ultrasound image data and measurement data. Further, in a case where the ultrasonic diagnostic device has a report producing function, the examination report may be produced in the ultrasonic diagnostic device.

Meanwhile, in a system described in Patent Literature 1, a server is synchronized with a workstation to manage medical images based on statuses thereof. In a device described in Patent Literature 2, an image interpretation report is reproduced based on information related to the image interpretation report.

### CITATION LIST

### PATENT LITERATURE

Patent Literature 1: JP 2000-268117 A
Patent Literature 2: JP 2013-137586 A

### SUMMARY

### TECHNICAL PROBLEM

When both devices of an ultrasonic diagnostic device and a workstation include a report producing function, a problem may arise in that the devices separately produce different examination reports for one examination. For example, after an examination report is produced in the workstation, an instruction to perform report production may be issued in the ultrasonic diagnostic device. In this case, regardless of the presence of the examination report produced in the workstation, a new examination report for the same examination as that associated with the examination report produced in the workstation will be independently produced in the ultrasonic diagnostic device. When the instruction to perform report production is issued in the workstation after production of an examination report in the ultrasonic diagnostic device, the same problem may arise.

An object of the present invention is to prevent production of a plurality of different examination reports associated with one examination. Another object of the present invention is to centrally manage an examination report.

### SOLUTION TO PROBLEM

An ultrasonic diagnostic device according to the present invention includes a report production unit that produces an examination report under a condition that duplicate production is restricted to prevent presence of multiple examination reports for one examination, a first manager unit that activates the report production unit upon receipt of an instruction to start report production from a user, and a second manager unit that causes the report production unit to produce the examination report when no examination report is produced upon completion of an examination.

According to the above-described configuration, even when the instruction to start report production is not given from the user, the examination report can be produced. In other words, the examination report whose production is not intended by the user is automatically produced. In the above-described configuration, duplicate production of examination reports for one examination is restricted. Because of this, once the examination report is produced for one examination, production of another examination report for the same examination is restricted. For example, other devices are restricted from producing a different examination report associated with the one examination. This can prevent a plurality of different examination reports being produced in connection with the one examination. When the instruction to start report production is provided from the user after the one examination is finished, for example, the previously produced examination report is retrieved rather than producing a new examination report. Then, the retrieved examination report is edited by the user.

The retrieved examination report may be edited to reflect observations (comments), or add an attachment of an ultrasound image. The ultrasound image to be attached may be a B-mode tomographic image, a three dimensional image, and an image including blood flow information.

Preferably, the ultrasonic diagnostic device further includes a transmission unit that transmits, when the examination report is produced, the produced examination report to an external device equipped with another report production unit, a transmission manager unit that manages each examination report in terms of whether or not the examination report is transmitted, a report acquisition unit that acquires, when an instruction to restart examination report production is given together with designation of a specific examination, the examination report associated with the specific examination, in which the examination report associated with the specific examination is acquired from the external device when that examination report has been transmitted, or retrieved within the ultrasonic diagnostic device when that examination report is not transmitted.

According to the above-described configuration, the examination report is automatically transmitted from the ultrasonic diagnostic device to the external device. The external device is a device having a report producing function. In this configuration, overlapped multiple examination reports are prevented from being produced in connection with one examination. Because of this, when the examination report is transmitted to the external device, the external device is prevented from producing another examination report. In this way, a plurality of different examination reports are prevented from being produced for the same examination in both the ultrasonic diagnostic device and the external device.. For example, when the instruction to start report production is issued to the external device from the user, the external device is caused to retrieve the examination report produced in the ultrasonic diagnostic device rather than producing a new examination report. Then, the retrieved examination report is edited by the user. Even when there is no examination report created upon completion of the examination, an examination report is automatically generated and transmitted to the external device. Accordingly, even in a case where the instruction to start report production is not given by the user in the ultrasonic diagnostic device, the external device is prevented from producing another examination report for the same examination.

Further, in the above-described configuration, a storage location of the examination report is identified based on a transmission status of the examination report, and the examination report is retrieved from the identified storage location. When the examination report has been transmitted to the external device, the examination report is acquired from the external device to which the examination report is transmitted. Even when the device itself stores the examination report, the transmitted examination report is acquired from the external device. This makes it possible to perform centralized management of the examination report. Specifically, when a particular examination report is produced in the ultrasonic diagnostic device, the produced particular examination report is automatically transmitted to the external device. Upon receipt of the instruction to restart production, the ultrasonic diagnostic device acquires the particular examination report from the external device. In this way, the examination report is centrally managed. For example, when the particular examination report is edited in the external device, the ultrasonic diagnostic device acquires the edited particular examination report from the external device. This allows the ultrasonic diagnostic device to acquire the most up-to-date examination report for one examination.

Preferably, in a case where the examination report associated with the specific examination is not retransmitted to the external device after the report acquisition unit has acquired the examination report associated with the specific examination, the external device is prevented from editing the examination report associated with the specific examination. According to this configuration, the external device cannot edit the examination report associated with the specific examination until the examination report associated with the specific examination is retransmitted to the external device. In other words, edit authority of the external device is restricted. In this way, the examination report is centrally managed.

Preferably, the report production unit has a function of reflecting measurement data obtained in an ongoing examination in the examination report.

Preferably, in operation to transmit the examination report, the transmission unit also transmits to the external device image data obtained in the examination associated with the examination report to be transmitted.

A method for producing an examination report according to the present invention includes a report production step of producing an examination report with a report producing function under a condition that duplicate production is restricted to prevent presence of multiple examination reports for one examination, a first management step of activating the report producing function in response to an instruction to start report production from a user, and a second management step of causing the report producing function to produce an examination report, when no examination report is produced upon completion of the examination,.

### ADVANTAGEOUS EFFECTS OF INVENTION

According to the present invention, a plurality of different examination reports are prevented from being produced for one examination. Alternatively, centralized management of the examination report can be achieved.

### BRIEF DESCRIPTION OF DRAWINGS

FIG. 1 is a block diagram showing an overall configuration of an ultrasonic diagnostic system according to an embodiment of this invention;
FIG. 2 is a diagram showing an example of a data management file;
FIG. 3 is a diagram showing an example of a tomographic image;
FIG. 4 is a diagram showing an example of a report editor screen;
FIG. 5 is an example of a screen for selecting an examination report;
FIG. 6 is a diagram showing an example of the examination report;
FIG. 7 is a flowchart showing an example of operation performed by the ultrasonic diagnostic device according to the embodiment;
FIG. 8 is a flowchart showing an example of operation performed by the ultrasonic diagnostic device according to the embodiment;
FIG. 9 is a diagram showing another example of the data management file, and
FIG. 10 is a diagram showing a further example of the data management file.

### DESCRIPTION OF EMBODIMENTS

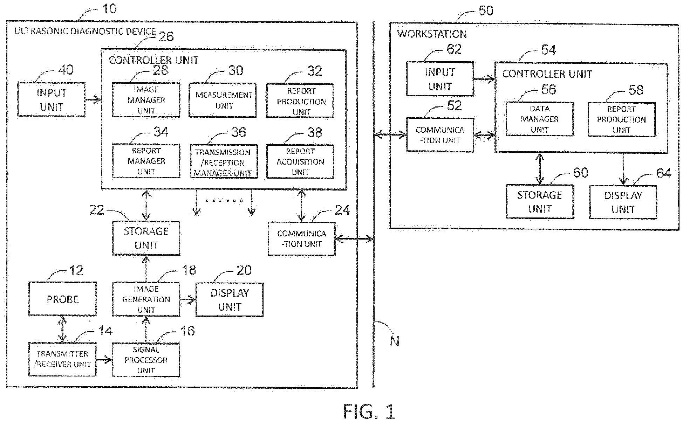

In FIG. 1, an embodiment of an ultrasonic diagnostic system according to the present invention is shown. FIG. 1 is a block diagram showing an overall configuration of the ultrasonic diagnostic system. The ultrasonic diagnostic system includes an ultrasonic diagnostic device 10 and a workstation 50. The ultrasonic diagnostic device 10 is connected through a communication channel N to the workstation 50. The communication channel N is a network, such as, for example, an in-hospital LAN.

The ultrasonic diagnostic device 10 is used in a medical field, and equipped with a function of transmitting/receiving ultrasound to generate an ultrasound image of an internal area of a living body. Further, the ultrasonic diagnostic device 10 also has functions of performing a variety of measurements, analyzing measurement data, producing an examination report, and editing the examination report. The examination report contains, for example, measurement data, analyzed results, observations (comments), an image, etc. The examination report is transmitted to an external device, such as the workstation 50.

The workstation 50 has functions of producing and editing the examination report. Further, the workstation 50 stores ultrasound image data generated by the ultrasonic diagnostic device 10. The workstation 50 may function as an image server.

Hereinafter, each component in the ultrasonic diagnostic device 10 will be described.

A probe 12 is a transducer for transmitting and receiving ultrasound. In the probe 12 equipped with a plurality of vibrating elements for transmitting and receiving ultrasound, an ultrasonic beam is formed by the plurality of vibrating elements. The ultrasonic beam is electronically scanned repeatedly. As an electronic scanning method, electronic sector scan, electronic linear scan, and the like have been known. For the probe 12, a 1D probe with a plurality of vibrating elements being arranged in series, or a 2D probe with a plurality of vibration elements being two dimensionally arranged, may be used.

A transmitter/receiver unit 14 functions as a transmission beam former and a reception beam former. In transmission, the transmitter/receiver unit 14 supplies the vibrating elements in the probe 12 with transmission signals which are delayed from each other by a fixed length of time. In this way, an ultrasound transmission beam is formed. In reception, the probe 12 receives ultrasound waves reflected from the living body, which causes the probe 12 to output a plurality of reception signals to the transmitter/receiver unit 14. In the transmitter/receiver unit 14, phased addition is performed on the plurality of reception signals, to output beam data as phased and added reception signals.

A signal processor unit 16 applies signal processing, such as detection, signal compression, gain adjustment, and filtering, to the beam data. The beam data obtained after the signal processing may be stored in a storage unit 22.

An image generator unit 18 is composed of a digital scan converter having a coordinate converting function, an interpolation processing function, and other functions. The image generator unit 18 generates ultrasound image data based on beam data obtained after the signal processing. For example, B mode tomographic image data is generated. The B mode tomographic image data is output to a display unit 20. As a result of this, the B mode tomographic image is displayed as a moving picture in real time.

In this embodiment, volume data may be obtained by scanning a three-dimensional space with the ultrasonic beam. In this case, the image generator unit 18 may perform three-dimensional image processing, such as volume rendering, on the volume data to generate three-dimensional image data. Further, a color blood flow image representing blood flow information and a Doppler waveform may be generated and displayed on the display unit 20. Still further, a plurality of images may be synthesized. For example, a controller unit 26 may combine the B-mode tomographic image with the color blood flow image and display the resulting combined image on the display unit 20.

The display unit 20 is composed of a display device, such as, for example, a liquid crystal display. The ultrasound image, an examination report, which will be described below, measurement data, which will be described below, and other data are displayed on the display unit 20.

The storage unit 22 is storage, such as a hard disk. The storage unit 22 stores the ultrasound image data, the examination report, the measurement data, etc.

A communication unit 24 is a communication interface connected to the communication channel N. The communication unit 24 has functions of transmitting data to another device and receiving data from the other device.

The controller unit 26 controls operation of each component in the ultrasonic diagnostic device 10. The controller unit 26 is connected to an input unit 40. The input unit 40 is composed of an operation panel, for example. The operation panel is a device including, for example, a keyboard, a trackball, and the like. A user can use the input unit 40 to input various types of information.

In addition, the controller unit 26 also includes an image manager unit 28, a measurement unit 30, a report production unit 32, a report manager unit 34, a transmission/reception manager unit 36, and a report acquisition unit 38.

The image manager unit 28 manages ultrasound image data generated by the image generator unit 18. For example, the image manager unit 28 manages the ultrasound image data on an examinee by examinee and an examination by examination basis. The image manager unit 28 associates the ultrasound image data with, for example, examinee identification information, examination identification information, and image identification information. The examinee identification information is information for identifying an examinee, and is composed of, for example, the name of the examinee, an examinee ID, and the like. The examination identification information is information for identifying an examination, and is composed of, for example, an examination ID. The image identification information is information for identifying ultrasound image data, and is composed of, for example, an image ID. As the examination identification information, for example, an ID unique to each examination (an examination unique ID: examination UID) is used. As image identification information, for example, an ID unique to each image (an image unique ID: image UID) is used. For example, IDs in accordance with DICOM standards are used. In addition, the ultrasound image data may be further associated with series identification information (such as, for example, a series ID) for identifying a series contained in the examination. The image manager unit 28 creates a data management file indicating a correlation among the examinee identification information, the examination identification information, and the image identification information. The data management file contains information (of a file path, such as, for example, a URL) indicative of a storage location of ultrasound image data. The data management file is stored in the storage unit 22. When a below-described examination report is produced, the produced examination report is associated with examination identification information. In this case, information for identifying the produced examination report is included in the data management file. It should be noted that the ultrasound image data may be stored in the workstation 50 and other external devices.

The measurement unit 30 performs various types of measurement using the ultrasound image data. For example, the measurement unit 30 has a function of measuring a distance between features, area of each of the features, or displacement of each of the features in the ultrasound image. In addition to these measurements, a value used for evaluating a heart function (such as, for example, a systolic output) may be measured. Other types of measurement may be conducted, of course. Further, the measurement unit 30 may analyze values obtained by measurement. Measurement data representing measured results and analyzed results is stored in the storage unit 22. The measurement data is associated, for example, with the examinee identification information, the examination identification information, and the image identification information.

The report production unit 32 has a function of producing an examination report. For example, the report production unit 32 has a function of displaying a report editor screen on the display unit 20. On the report editor screen, the user can input or edit observations (comments), measurement data, and other values using the input unit 40. The report production unit 32 may retrieve from the storage unit 22 measurement data obtained in the examination to include the retrieved measurement data in the examination report. Further, using the input unit 40, the user can attach or delete an ultrasound image to or from the examination report. When a particular ultrasound image is attached to the examination report, the report production unit 32 enters the image UID of data of the particular ultrasound image at a predetermined position in the examination report. Alternatively, the report production unit 32 may enter information (of the file path, such as a URL, for example) indicative of the storage location of data of the particular ultrasound image at a predetermined position in the examination report. Further alternatively, the report production unit 32 may directly embed data of the particular ultrasound image itself at a predetermined position in the examination report. The report production unit 32 displays, for example, a list of ultrasound images obtained in the examination on the display unit 20, and attaches to the examination report an ultrasound image selected from the list by the user.

The examination report is stored in the storage unit 22. The report production unit 32 manages examination reports on an examinee by examinee and an examination by examination basis. For example, the report production unit 32 associates each of the examination reports with the examinee identification information, the examination identification information, and report identification information. The report identification information is information for identifying an examination report, and is composed of, for example, a report ID. The report production unit 32 adds, for example, the report identification information into the data management file.

In this embodiment, production of the examination report is restricted per examination to prevent the presence of different examination reports for one examination. Whether or not the examination report about a particular examination is produced can be determined, for example, by referring to the data management file. When the examination report is produced as described above, report identification information of the produced examination report is added into the data management file. When an instruction to start report production is given with designation of a specific examination from the user, it can be determined by referring to the data management file whether or not an examination report associated with the designated specific examination has already been produced. The determination is conducted by the controller unit 26, for example. When the data management file includes no report identification information of the examination report associated with the designated specific examination, the report production unit 32 produces a new examination report for the designated specific examination. On the other hand, when the data management file includes report identification information of the examination report associated with the designated specific examination, the examination report which is previously produced is acquired. In this case, production of a new examination report for the designated specific examination is prevented. As described below, when an examination report is produced, the produced examination report is automatically transmitted to the workstation 50 and stored in the workstation 50. In this case, the produced examination report is acquired from the workstation 50. Meanwhile, in a case where the produced examination report is not transmitted to the workstation 50 due to transmission error or other failures, the produced examination report is acquired from the storage unit 22 in the ultrasonic diagnostic device 10. An object that may be edited is the examination report acquired from the workstation 50 or the storage unit 22. It should be noted that operation to acquire the examination report is performed by the report acquisition unit 38 which will be described below.

Another technique may be used to prevent production of an examination report. For example, in the ultrasonic diagnostic device 10 and the workstation 50, the examination report associated with a specific examination designated by the user is searched for, and production of a new examination report for the specific examination may be allowed when the examination report associated with the specific examination is not found. This search is performed, for example, by the report acquisition unit 38. On the other hand, when the examination report being searched for is found to be stored in the workstation 50, the report acquisition unit 38 acquires the examination report from the workstation 50. When the examination report being searched for is not transmitted to the workstation 50, the report acquisition unit 38 acquires the examination report from the storage unit 22. The thus-acquired examination report can be subjected to editing operations.

Alternatively, the report production unit 32 itself may have the function of preventing duplicate production to prevent the presence of different examination reports for one examination. For example, the report production unit 32 may manage examinations (examination UIDs) associated with previously produced examination reports, and prevent, when the instruction to produce a report is given from the user, production of multiple examination reports associated with one examination (examination UID).

Further, the report production unit 32 may create an image list file for identifying an ultrasound image attached to the examination report. The image list file includes the image UID of the ultrasound image attached to the examination report and information (of the file path, for example) indicative of the storage location of data of the attached ultrasound image. When a particular ultrasound image is added to the examination report, the image UID and the file path of data of the particular ultrasound image are included into the image list file. When the particular ultrasound image is deleted from the examination report, the image UID and file path of the particular ultrasound image are accordingly deleted from the image list file. The ultrasound image attached to the examination report and its storage location can be identified by referring to the image list file.

Still further, the report production unit 32 may have a function of converting a data format of the examination report into an output data format and a function of displaying the converted examination report on the display unit 20. The output data format is, for example, a data format for printing, and specifically, the examination report is PDF (Portable Document Format) data. When the image UID is described in the examination report, the report production unit 32 refers to the data management file stored in the storage unit 22 to identify the file path associated with the recorded image UID, and acquires ultrasound image data associated with the image UID from the storage location indicated by the identified file path. The report production unit 32 embeds the acquired ultrasound image data at a predetermined position in the examination report and converts the data format of the examination report embedded with the ultrasound image data into the output data format. Alternatively, when the file path is described in the examination report, the report production unit 32 may acquire ultrasound image data from the storage location indicated by the described file path and embed the acquired ultrasound image data at the predetermined position in the examination report. Further alternatively, the report production unit 32 may refer to the image list file to identify the file path of the ultrasound image attached to the examination report and acquire data of the attached ultrasound image from the storage location indicated by the identified file path.

The report manager unit 34 manages examination report production performed by the report production unit 32. For example, when the instruction to start report production is given from the user, the report manager unit 34 activates the report production unit 32. On the other hand, when an instruction to finish report production is given from the user, the report manager unit 34 controls the report production unit 32 to produce the examination report that includes the contents having been entered up until the instruction was given. The produced examination report is stored in the storage unit 22.

Meanwhile, in a case where no examination report is produced upon completion of an examination, the report manager unit 34 activates the report production unit 32 and instructs the report production unit 32 to produce an examination report. This ensures the presence of the produced examination report even when no instruction to start report production is given from the user until the examination is finished. When measurement data is obtained, the obtained measurement data may be reflected on the examination report. Further, when a particular ultrasound image is viewed by the user, the particular ultrasound image being viewed may be attached to the examination report. In this way, the examination report is automatically produced even in the absence of the instruction from the user.

In this embodiment, after the examination is finished, the communication unit 24 automatically transmits an examination report about the finished examination together with ultrasound image data and measurement data obtained through the examination. Regardless of whether the examination report is produced in accordance with the instruction from the user or produced in the absence of the instruction from the user, the produced examination report is automatically transmitted to the workstation 50. As a result, the produced examination report is stored in the workstation 50. In the workstation 50, when the instruction to start report production is provided from the user, the examination report associated with the examination designated by the user can be edited as long as that report is stored in the workstation 50. In this case, a new examination report is prohibited from being produced for the designated examination.

Data of all ultrasound images obtained in the examination may be transmitted to the workstation 50, or data of some of the ultrasound images obtained in the examination (for example, data of the ultrasound images attached to the examination report) may be transmitted to the workstation 50. In this way, the workstation 50 stores the examination report for the examination, and data of both the ultrasound images and the measurements obtained in the examination. Further, the data management file and the image list file may be transmitted to the workstation 50. The communication unit 24 may establish communication in accordance with DICOM standards or in accordance with HTTP.

The transmission/reception manager unit 36 manages each examination report in terms of whether or not the examination report is transmitted to the workstation 50. The transmission/reception manager unit 36 creates a transmission/reception status management file indicative of management details. The transmission/reception status management file is stored in the storage unit 22. For example, there may be a case where transmission error occurs due to a fault in the communication path, resulting in failure of transmission of the examination report to the workstation 50. In this case, the transmission status of the examination report is set to "not-transmitted". The transmission/reception manager unit 36 periodically or continuously monitors the examination report whose transmission state is specified as "not-transmitted". Any examination report whose transmission state is specified as "not-transmitted" is flagged as an object of a transmission retry. If this is the case, the communication unit 24 repeatedly performs operation to transmit data (including the not-transmitted examination report, and data of the ultrasound images and measurements obtained in the examination associated with the not-transmitted examination report). Upon completion of the transmission, the transmission status is specified as "transmitted".

The report acquisition unit 38 acquires, when an instruction to restart examination report production is issued together with designation of a specific examination from the user, the examination report associated with the designated specific examination. The report acquisition unit 38 acquires that examination report from a storage location corresponding to the transmission status of the examination report. The report acquisition unit 38 refers, for example, to the transmission/reception status management file to identify the transmission status. When the examination report associated with the designated specific examination has been transmitted, the report acquisition unit 38 acquires via the communication unit 24 the examination report associated with the designated specific examination from the workstation 50. The communication unit 24 fetches the examination report through HTTP communication or DICOM communication, for example. When the examination report associated with the designated specific examination is not transmitted, the report acquisition unit 38 acquires the examination report from the storage unit 22.

The examination report acquired by the report acquisition unit 38 is sent to the report production unit 32. The report production unit 32 displays the report editor screen on the display unit 20. On the report editor screen, the user can edit the acquired examination report using the input unit 40. Meanwhile, the report production unit 32 refers to the data management file to acquire data of ultrasound images associated with the specific examination designated by the user, and displays a list of the ultrasound images on the display unit 20. Then, edit and other operations are performed on the examination report by the user. In the edit operation, the observations (comments) contained in the examination report may be edited or supplied with additional comments, another ultrasound image may be added to the examination report, one or more of the ultrasound images in the examination report may be deleted, and/or measurement data in the examination report may be edited or deleted. The edited examination report is stored in the storage unit 22. This causes the examination report associated with the specific examination to be updated with the edited examination report in the storage unit 22. In addition, the edited examination report is automatically transmitted via the communication unit 24 to the workstation 50. This causes the examination report associated with the specific examination to be updated with the edited examination report in the workstation 50. In addition, the transmission status of the edited examination report is managed by the transmission/reception manager unit 36.

Next, each component in the workstation 50 will be described.

A communication unit 52 is a communication interface connected to the communication path N. The communication unit 52 has a function of transmitting data to another device and a function of receiving data from the other device. In this embodiment, when an examination is finished, the communication unit 52 receives an examination report for the finished examination together with ultrasound image data and measurement data obtained in the finished examination from the ultrasonic diagnostic device 10. Further, the communication unit 52 may receive a data management file related to the finished examination from the ultrasonic diagnostic device 10.

When the examination report associated with the specific examination designated in the ultrasonic diagnostic device 10 has been transmitted from the ultrasonic diagnostic device 10 to the workstation 50, the communication unit 52 acquires the examination report associated with the specific examination from a storage unit 60 and transmits the acquired examination report to the ultrasound diagnostic device 10. Here, the communication unit 52 may transmit the ultrasound image data and the measurement data associated with the specific examination to the ultrasonic diagnostic device 10, or transmit data of only those attached to the examination report to the ultrasonic diagnostic device 10.

A controller unit 54 controls each of the components in the workstation 50. In addition, the controller unit 54 is connected to an input unit 62. The input unit 62 is a device equipped with a keyboard, a trackball, or the like. The user can use the input unit 62 to input various types of information.

The controller unit 54 further includes a data manager unit 56 and a report production unit 58.

The data manager unit 56 causes the storage unit 60 to store the ultrasound image data, the measurement data, and the examination report received through the communication unit 52. The data manager unit 56 manages the ultrasound image data, the measurement data, and the examination report on an examinee by examinee and examination by examination basis. The data manager unit 56 creates a data management file for indicating, for example, correlation among the examinee identification information, the examination identification information, the image identification information, and the report identification information, and manages data based on the data management file. The data management file contains information (of the file path, such as, for example, a URL) indicative of the storage location of the ultrasound image data. The data manager unit 56 may use the data management file received from the ultrasonic diagnostic device 10 to manage data. The data management file is stored in the storage unit 60.

The report production unit 58 has the function of producing an examination report. In the workstation 50, when the instruction to start examination report production is received with designation of a specific examination from the user, the report production unit 58 acquires the examination report associated with the specific examination from the storage unit 60 as long as that examination report is stored in the storage unit 60. The report production unit 58 displays on a display unit 64 the report editor screen on which the acquired examination report can be edited. On the report editor screen, the user can edit the examination report using an input unit 62. For example, the user can perform operations to add or edit observations (comments), include or remove an ultrasound image, and edit or delete measurement data, etc. For example, the report production unit 58 refers to the data management file to acquire data of ultrasound images associated with the specific examination designated by the user, and displays a list of the ultrasound images on the display unit 64. The report production unit 58 adds to the examination report the ultrasound image selected from the list by the user. The report production unit 58 describes an image UID or a file path of data of the added ultrasound image at the predetermined position in the examination report. Upon addition of the ultrasound image to the examination report, the image UID and the file path of the added ultrasound image are included in an image list file. When a particular ultrasound image is deleted from the examination report, the image UID and the file path of the deleted particular ultrasound image are eliminated from the image list file. The resulting examination report edited by the report production unit 58 is stored in the storage unit 60. Then, in the storage unit 60, the examination report associated with the specific examination is updated with the edited examination report.

The report production unit 58 has a function of producing a new examination report. In this embodiment, as in the case of the above-described report production unit 32, production of different examination reports for one examination is also prevented in the report production unit 58. Production of different examination reports may be prevented in a manner similar to the report production unit 32, for example, by referring to the data management file, including a function of restricting production of an examination report in the report production unit 58 itself, or using a result of searching for the examination report.

In this embodiment, when a particular examination is conducted with the ultrasonic diagnostic device 10, an examination report for the particular examination is automatically produced in the ultrasonic diagnostic device 10 even in the absence of the instruction to start report production received from the user. The produced examination report is automatically transmitted to the workstation 50 and stored therein. When the particular examination conducted with the ultrasonic diagnostic device 10 is designated in the workstation 50, the workstation 50 retrieves the examination report associated with the designated particular examination from the storage unit 60 rather than producing a new examination report. Then, the retrieved examination report works as an object to be edited.

The report production unit 58 has the function of converting a data format of the examination report into the output data format and the function of displaying the converted examination report on the display unit 64. When the image UID is described in the displayed examination report, the report production unit 58 refers to the data management file stored in the storage unit 60 to identify a file path corresponding to the described image UID. Then, the report production unit 58 acquires data of the ultrasound image associated with the image UID from the storage location indicated by the identified file path. The report production unit 58 embeds acquired data of the ultrasound image at the predetermined position in the examination report, and converts the data format of the examination report embedded with ultrasound image data into the output data format. On the other hand, when the file path is described in the displayed examination report, the report production unit 58 may acquire data of the ultrasound image from the storage location indicated by the described file path, and may embed acquired data of the ultrasound image at the predetermined position in the examination report. Alternatively, the report production unit 58 may refer to the image list file to identify the file path of the ultrasound image attached to the displayed examination report, and acquire data of the attached ultrasound image from the storage location indicated by the identified file path.

The storage unit 60 is a storage device such as a hard disc. The storage unit 60 stores the ultrasound image data, the examination report, the measurement data, and others.

The display unit 64 is composed of a display device such as, for example, a liquid crystal display. The display unit 64 displays the ultrasound image, the measurement data, the examination report, and other items.

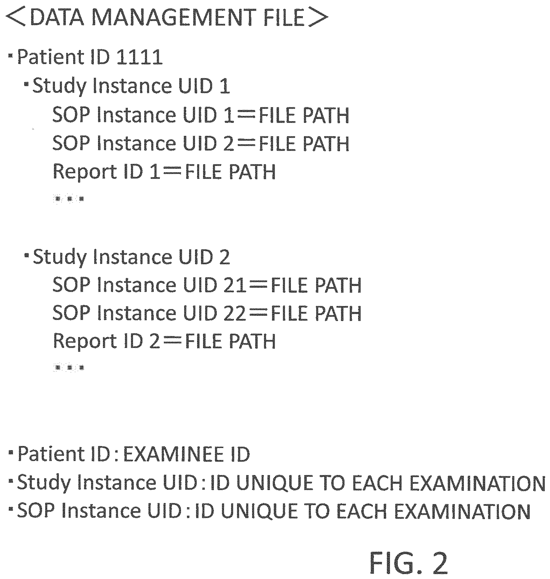

FIG. 2 shows an example of the data management file. The data management file may be stored in either or both of the ultrasonic diagnostic device 10 and the workstation 50.

Reference letters "Patient ID" represent an examinee ID. Reference letters "Study Instance UID" represent an ID unique to each examination (examination UID). Reference letters "SOP Instance UID" represent an ID unique to each image (image UID). Reference letters "Report ID" represent a report ID. The IDs are associated with each other. Further, the image UID and the report ID are associated with the file path (information representing the storage location). In the data management file, the examination ID, the image ID, and the report ID are associated with each examinee ID. When the data management file is referred to, examinations can be identified for each examinee, and storage locations of ultrasound image data and the examination report can be identified for each examination. In addition, the data management file may also contain both identification information and storage location information of the measurement data.

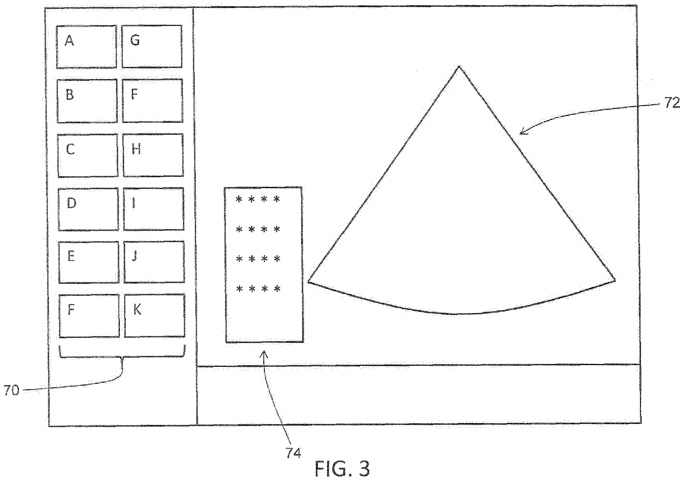

In FIG. 3, an example of an ultrasound image is illustrated. FIG. 3 shows a screen viewed in a measurement mode. When the user, such as a medical operator, provides an instruction to start a new examination with the input unit 40, for example, ultrasonic waves are transmitted and received to generate the ultrasound image. The generated ultrasound image is displayed on the display unit 20. It should be noted that before starting the examination, information about the examinee, including the examinee ID, is input in the ultrasonic diagnostic device 10. Data of the ultrasound image is stored in the storage unit 22. The examinee ID, the examination UID, and the image UID are correlated to each other, and the data management file indicating the correlation among the examinee ID, the examination and image UIDs is created.

Then, in response to an instruction to start measurement provided by the user through the input unit 40, the mode of the ultrasonic diagnostic device 10 is changed to the measurement mode. In the measurement mode, a list of ultrasound images obtained in an ongoing examination (the ultrasound images associated with the examination UID of the ongoing examination) is shown on the display unit 20. In the example shown in FIG. 3, a list 70 of thumbnails (reduced images) of the ultrasound images obtained in the ongoing examination is displayed on the display unit 20. The displayed list 70 includes, for example, thumbnails of ultrasound images A to K. For example, the controller unit 26 generates the thumbnails and displays the generated thumbnails on the display unit 20. The user can use the input unit 40 to select an ultrasound image to be attached to the examination report from the list 70. When the user selects, for example, the ultrasound images A, B, and C, the ultrasound images A, B, and C are attached to the examination report. The report production unit 32 enters each image UID or storage location information of the ultrasound images A, B, and C in the examination report.

On the other hand, when the user selects from the list 70 an ultrasound image to be measured, the controller unit 26 enlarges the selected ultrasound image to be measured and displays the enlarged ultrasound image on the display unit 20. For example, a B mode tomographic image 72 is an image selected by the user as the image to be measured. When the user provides an instruction to perform measurement through the input unit 40, the measurement unit 30 performs the instructed measurement on the B mode tomographic image 72. The controller unit 26 displays measurement data 74 indicative of measured results on the display unit 20. The measurement data 74 is stored in the storage unit 22. The measurement data 74, an evaluation value based on the measurement data 74, and other values are reflected in the examination report. Here, an image obtained by merging the B mode tomographic image with a color blood flow image, a three-dimensional image, or a Doppler waveform may be displayed.

When the user provides the instruction to start examination report production using the input unit 40 after the completion of measurement, the report manager unit 34 activates the report production unit 32. The report production unit 32 displays the report editor screen on the display unit 20. On the report edit screen, the user can check and edit examination data, input comments, and perform other operations.

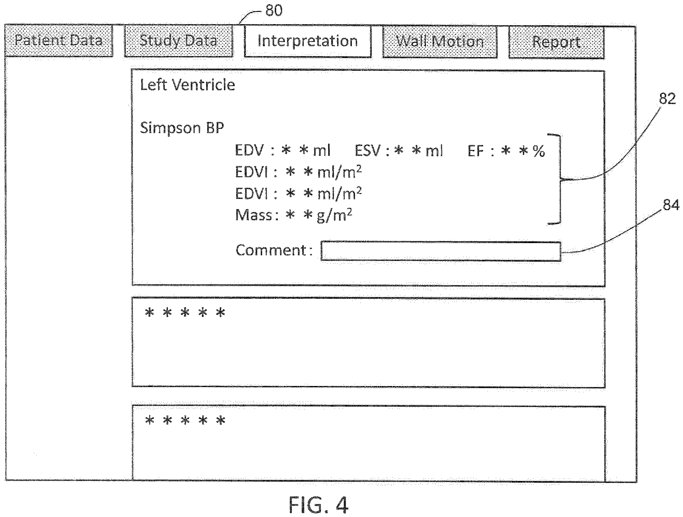

FIG. 4 shows an example of the report editor screen. A report editor screen 80 contains a measurement data field 82 and a comment input field 84. The report production unit 32 acquires from the storage unit 22 the measurement data obtained by the measurement unit 30, and enters the acquired measurement data in the measurement data field 82. On this screen, the user can check the measurement data. Using the input unit 40, the user can edit the measurement data. In addition, it may be configured that when the user edits measurement data, the measurement data is displayed with a specific color. This allows the user to recognize a situation where the measurement data is edited. Further, the user can use the input unit 40 to input observations (comments) in the comment input field 84.

When the user issues, through the input unit 40, an instruction to complete production of the examination report, the report production unit 32 produces the examination report whose contents include data having been entered or edited at the time of issuance of the instruction. The produced examination report is stored in the storage unit 22. The examinee ID, the examination UID, the image UID, and the report ID are correlated to each other, and the data management file indicating their correlation is created.

Then, when the user issues the instruction to start measurement through the input unit 40, the mode of the ultrasonic diagnostic device 10 is changed to the measurement mode again. In this case, as shown in FIG. 3, the list 70 of ultrasound images acquired in the present examination is displayed on the display unit 20. Using the input unit 40, the user can select from the list 70 an ultrasound image to be attached to the examination report. The selected ultrasound image is attached to the examination report. Further, when the user provides an instruction to conduct remeasurement or new measurement, the measurement unit 30 executes the instructed remeasurement or new measurement. Obtained measurement data is reflected in the examination report.

When the user issues the instruction to start production of the examination report using the input unit 40 after the completion of measurement, the report manager unit 34 activates the report production unit 32. The report production unit 32 acquires the examination report for the present examination (the examination report associated with the examination UID of the present examination) stored in the storage unit 22. As shown in FIG. 4, the report production unit 32 displays the report editor screen 80 on the display unit 20. The report production unit 32 merges the contents of the previously produced examination report with the measurement data obtained in the remeasurement, and displays the merged result on the report editor screen 80. That is, when items are remeasured, the report production unit 32 fills each field of the items with measurement data obtained by the remeasurement. On the other hand, for items which are not remeasured, the report production unit 32 fills each field of the not-remeasured items with data from the contents of the previously produced examination report. Further, when there is a newly measured item, the report production unit 32 fills a field of the item with measurement data obtained in the new measurement. Meanwhile, the report production unit 32 fills the comment input field 84 with the previously input observations (comments). In this way, the fields of the not-remeasured items and the previous observations (comments) are filled with data reproduced from the previous contents, while the fields of the remeasured items and newly measured items are filled with presently obtained measurement data. As has been described above with reference to FIG. 4, the user can edit the measurement data and the observations (comments).

When the user issues the instruction to complete examination report production through the input unit 40, the report production unit 32 produces the examination report whose contents include data having been filled or edited at the time of issuance of the instruction. The produced examination report is stored in the storage unit 22. This causes the examination report to be updated.

Then, the present examination is finished when the user provides an instruction to finish the examination. In this case, the communication unit 24 automatically transmits the examination report for the present examination together with ultrasound image data and measurement data obtained in the present examination to the workstation 50. As a result of this transmission, the examination report, ultrasound image data, and measurement data are stored in the storage unit 60 of the workstation 50. Further, the communication unit 24 may also transmit the data management file to the workstation 50.

In a case where the user issues no instruction to start report production until the examination is finished, the report manager unit 34 activates the report production unit 32. The report production unit 32 automatically produces the examination report. When measurement data has been obtained, the report production unit 32 produces the examination report in which the obtained measurement data is reflected. Then, the communication unit 24 automatically transmits the produced examination report as well as the ultrasound image data and measurement data obtained in the present examination to the workstation 50. In this way, the examination report, ultrasound image data, and examination data are stored in the storage unit 60 of the workstation 50. In addition, the communication unit 24 may also transmit the data management file to the workstation 50.

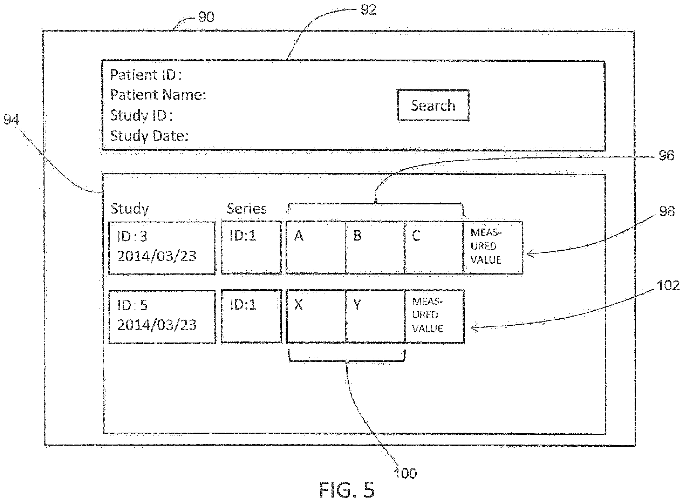

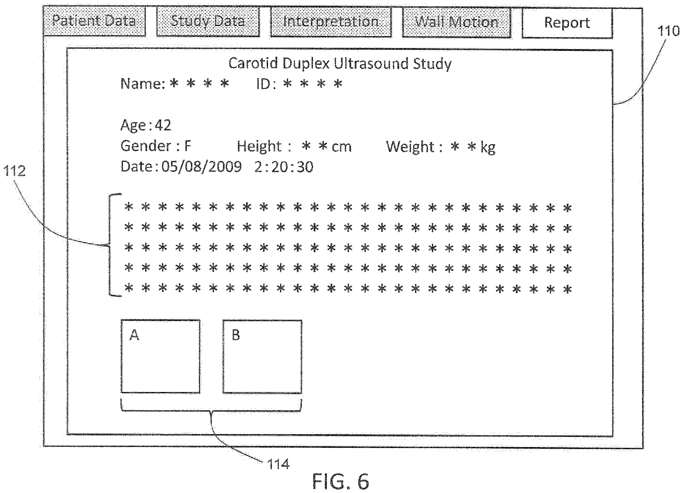

Next, referring to FIGS. 5 and 6, process steps for editing the examination report for a past examination will be described. In the ultrasonic diagnostic device 10, when an instruction to search for an examination report is received through the input unit 40 from the user, the report production unit 32 displays an examination report search screen on the display unit 20. FIG. 5 shows an example of such a search screen. A search screen 90 contains a search condition input field 92 and a searched result field 94. The input field 92 includes an examinee ID input field, an examinee name input field, an examination ID input field, and an examination date input field. When the user instructs a search after inputting search conditions, such as the examinee ID, the report production unit 32 refers to the data management file to retrieve from the storage unit 22 ultrasound image data and measurement data that meet the search conditions. The report production unit 32 displays searched results on the display unit 20. In the example shown in FIG. 5, data having an examination ID "3" and data having an examination ID "5" are displayed as the searched results. The displayed data is associated with a specific examinee ID. An ultrasound image data group 96 and a measurement data file 98 are associated with both the examination ID "3" and a series ID "1". An ultrasound image data group 100 and a measurement data file 102 are associated with the examination ID "5" and the series ID "1". The ultrasound image data groups 96 and 100 are displayed as thumbnails, for example. The measurement data files 98 and 102 are files in which measurement data is described. For example, ultrasound images A, B, and C are associated with both the examination ID "3" and the series ID "1". Ultrasound images X and Y are associated with both the examination ID "5" and the series ID "1". When the user selects an ultrasound image from the ultrasound image data group, the selected ultrasound image is enlarged and displayed on the display unit 20. For example, in response to selection of the ultrasound image A by the user, the ultrasound image A is enlarged and displayed on the display unit 20. Further, in response to selection of the measurement data file, selected measurement data is displayed on the display unit 20.

When the user designates a particular ultrasound image, the image UID of the selected particular ultrasound image is identified. The image UID is associated with the examination UID. Accordingly, designation of the particular ultrasound image by the user leads to designation of the examination UID.

Further, when the user issues the instruction to start report production in addition to designating an ultrasound image, the report acquisition unit 38 refers to the data management file to identify the examination UID associated with data of the ultrasound image designated by the user, and acquires the examination report associated with the identified examination UID. The report acquisition unit 38 refers to a transmission/reception status management file to identify a transmission status of the examination report associated with the identified examination UID. When the examination report has been transmitted, the report acquisition unit 38 acquires the examination report through the communication unit 24 from the workstation 50. When the examination report is not transmitted, the report acquisition unit 38 acquires the examination report from the storage unit 22. For example, in the example shown in FIG. 5, it is assumed that the ultrasound image A is designated by the user. The designation leads to designation of the examination ID "3". When the examination report associated with the examination ID "3" has been transmitted, the report acquisition unit 38 acquires the transmitted examination report from the workstation 50. When the examination report associated with the examination ID "3" is not transmitted, the report acquisition unit 38 acquires the not-transmitted examination report from the storage unit 22.

Then, as shown in FIG. 4, the report production unit 32 displays the report editor screen 80 on the display unit 20. The report production unit 32 enters data of the examination report acquired by the report acquisition unit 38 into each item on the report editor screen 80. This reproduces the contents of the previously produced examination report. On the report editor screen 80, the user is able to edit the measurement data and the observations (comments), and also able to add ultrasound image data to the examination report. When the ultrasound image data is added to the examination report, the report production unit 32 acquires from the storage unit 22 the ultrasound image data associated with the designated examination. Instead, the report production unit 32 may acquire through the communication unit 24 from the workstation 50 the ultrasound image data associated with the designated examination. For example, the ultrasound image data group associated with the examination ID "3" is acquired and displayed on the display unit 20. Data of one or more ultrasound images selected from the displayed ultrasound image data group by the user is included in the examination report.

When the user issues the instruction to complete examination report production using the input unit 40, the report production unit 32 produces the examination report whose contents include data having been entered or edited at the time of issuance of the instruction. The produced examination report is stored in the storage unit 22, which causes the examination report associated with the examination ID "3" to be updated. In addition, the communication unit 24 automatically transmits the produced examination report to the workstation 50. As a result of the transmission, the examination report is stored in the storage unit 60 of the workstation 50, and the examination report associated with the examination ID "3" is accordingly updated.

Meanwhile, when the user provides an instruction to authorize finalization through the input unit 40, the report production unit 32 converts the data format of the examination report into the output data format. The examination report is converted, for example, into PDF data, and the converted data is displayed on the display unit 20.

FIG. 6 shows a converted examination report. The report production unit 32 displays a converted examination report 110 onto the display unit 20. The examination report 110 contains observations (comments) 112 and ultrasound images 114 which are composed of information input or designated by the user. The report production unit 32 refers to the data management file stored in the storage unit 22 to identify the file path associated with the image UID described in the examination report. In the example shown in FIG. 6, file paths of the ultrasound images 114 are identified. The report production unit 32 acquires data of the ultrasound images 114 from storage locations directed by the identified file paths, and embeds the ultrasound images 114 at the designated position in the examination report. Data of the examination report 110 may be stored in the storage unit 22, transmitted to external devices, or printed with a printer.

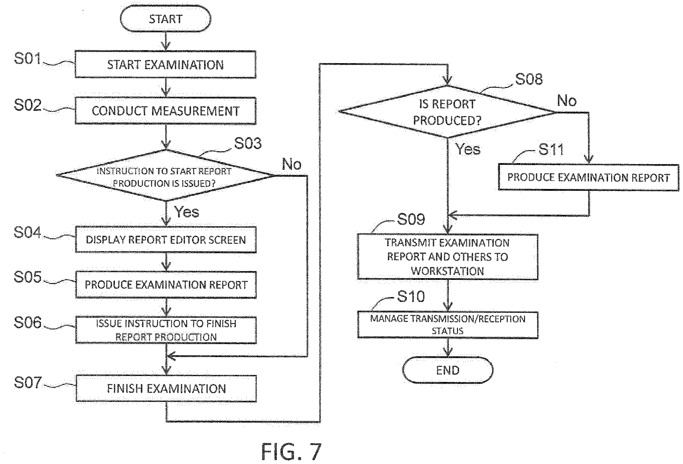

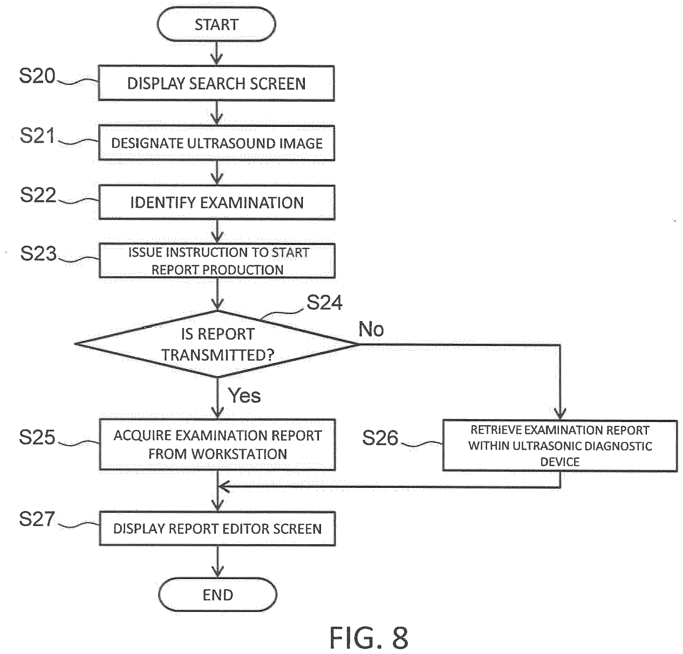

Next, operation of the ultrasonic diagnostic device 10 will be described with reference to flowcharts shown in FIGS. 7 and 8. First, referring to FIG. 7, process steps to produce the examination report are explained.

Initially, in the ultrasonic diagnostic device 10, the user issues an instruction to start an examination through the input unit 40 (S01). In response to the instruction, ultrasound is transmitted and received to generate data of an ultrasound image which is displayed on the display unit 20. Then, the examinee ID, the examination UID, and the image UID are correlated with each other, and a data management file for indicating their correlation is generated. The ultrasound image data and the data management file are stored in the storage unit 22.

Next, when the user issues a measurement instruction through the input unit 40, the mode of the ultrasonic diagnostic device 10 is shifted to the measurement mode (S02). For example, as shown in FIG. 3, the B mode tomographic image 72, the list 70 of ultrasound images, and the measurement data 74 are displayed on the display unit 20, on which the user may select one or more ultrasound images to be attached to the examination report by means of the input unit 40.

When the user provides the instruction to start report production using the input unit 40 (YES in S03), the report production unit 32 displays, as shown in FIG. 4, the report editor screen 80 on the display unit 20 (S04). Using the input unit 40, the user inputs the observations (comments) and other data. In this way, the examination report is prepared (S05). On the other hand, when the instruction to start report production is not provided (NO in S03), operation moves to a process in S07.

When the user issues the instruction to finish report production through the input unit 40 (S06), the report production unit 32 produces the examination report whose contents include data having been entered at the time of issuance of the instruction. The produced examination report is stored in the storage unit 22. Further, the examinee ID, the examination UID, the image UID, and the report ID are correlated with each other, and the data management file for indicating their correlation is generated. The data management file is stored in the storage unit 22.

Here, when the user issues the measurement instruction again, the mode of the ultrasonic diagnostic device 10 is shifted again to the measurement mode to conduct measurement. Further, when the user issues the instruction to start report production again, the report editor screen 80 is redisplayed on the display unit 20. At this time, the contents of the previously produced examination report are merged with measurement data obtained by the present measurement, and merged results are displayed on the report editor screen 80. Then, in response to the instruction to finish report production issued by the user, an examination report whose contents include data having been entered or edited at the time of issuance of the instruction is produced. The produced examination report is stored in the storage unit 22, which causes the examination report to be updated with the newly produced examination report.

Next, when the user issues the instruction to finish the examination through the input unit 40 (S07), whether or not the examination report for the present examination is produced is determined (S08).

When the examination report for the present examination is produced and stored in the storage unit 22 (YES in S08), the communication unit 24 automatically transmits the examination report for the present examination as well as the ultrasound image data and measurement data obtained in the present examination to the workstation 50 (S09). The communication unit 24 may transmit the data management file to the workstation 50. When transmission is complete, the transmission/reception manager unit 36 sets the status of "transmitted" to the transmission/reception status of the examination report for the present examination. When transmission is not completed, the transmission/reception manager unit 36 sets the status of the transmission/reception status of the examination report which is not transmitted to "not-transmitted". The transmission/reception manager unit 36 produces a transmission/reception status management file for representing the transmission/reception status of the examination report (S10). The produced transmission/reception status management file is stored in the storage unit 22.

When the examination report for the present examination is not produced (NO, in S08), i.e. when the examination report for the present examination is not stored in the storage unit 22, the report production unit 32 automatically produces the examination report (S 11). Then, the communication unit 24 transmits the produced examination report as well as the ultrasound image data and measurement data obtained in the present examination to the workstation 50 (S09). When transmission is complete, the transmission/reception status of the examination report for the present examination is specified as "transmitted". In a case where the transmission is incomplete, the transmission/reception status of the examination report is specified as "not-transmitted". The transmission/reception management file for representing the specified transmission/reception status is produced and stored in the storage unit 22 (S10).

With the above-described process steps, the examination report for the present examination and the ultrasound image data and measurement data obtained in the present examination are automatically transmitted to the workstation 50. Even when no instruction to start report production is issued from the user, the examination report is automatically produced and automatically transmitted to the workstation 50.

Next, process steps to acquire the examination report are described with reference to FIG. 8. Initially, in the ultrasonic diagnostic device 10, when the user issues the instruction to search for an examination report through the input unit 40, as shown in FIG. 5, the report production unit 32 displays the examination report search screen on the display unit 20 (S20). On the displayed screen, the user designates a specific ultrasound image through the input unit 40 (S21). The report production unit 32 identifies the examination associated with the designated specific ultrasound image (S22). Because the image UID of an ultrasound image is associated with the examination UID, designation of the ultrasound image leads to identification of the examination UID that is associated with the image UID of the designated ultrasound image. Then, the examination associated with the ultrasound image designated by the user is identified.

When the user issues the instruction to start report production through the input unit 40 (S23), whether or not the examination report assigned with the identified examination UID is transmitted to the workstation 50 is determined (S24). The report acquisition unit 38 refers to the transmission/reception status management file to find the transmission/reception status of the examination report associated with the identified examination UID. When the examination report is found to be transmitted (YES in S24), the report acquisition unit 38 acquires the examination report via the communication unit 24 from the workstation 50 (S25). On the other hand, when the examination report is found to be untransmitted (NO, in S24), the report acquisition unit 38 acquires the examination report from the storage unit 22 in the ultrasound diagnostic device 10 (S26).

Then, as shown in FIG. 4, the report production unit 32 displays the report editor screen 80 on the display unit 20 (S27). The report editor screen 80 reflects the contents of the examination report acquired by the report acquisition unit 38. In this way, the contents of the examination report obtained in the last production are reproduced.

Subsequent to this, the user can edit the examination report. The observations and measurement data may be edited; or addition or deletion of the ultrasound image may be performed. For example, the report production unit 32 refers to the data management file to acquire the ultrasound image data group associated with the designated examination UID from the storage unit 22 or the workstation 50. The report production unit 32 displays thumbnails of the ultrasound images on the display unit 20. One of the ultrasound images selected from the thumbnails by the user is added to the examination report. When the user provides the instruction to finish report production, an examination report whose contents include data having been entered or edited up until the instruction was provided is produced. The produced examination report is stored in the storage unit 22 and automatically transmitted to the workstation 50. This causes the ultrasonic diagnostic device 10 and the workstation 50 to update the examination report associated with the designated examination UID with the newly produced examination report.

As described above, according to this embodiment, in a case where no examination report is produced when the examination is finished, the examination report is automatically produced. In other words, the examination report is automatically produced even without the instruction to start report production issued by the user. After the completion of the examination, when the user issues the instruction to start report production together with designation of a specific examination, the previously produced examination data is retrieved rather than producing a new examination report for the designated specific examination. The retrieved examination report is the object to be edited. In this way, the examination report is automatically produced in the ultrasonic diagnostic device 10 and used as the object to be edited, which can prevent production of different examination reports for one examination.

In addition, the examination report is automatically transmitted from the ultrasonic diagnostic device 10 to the workstation 50. In this embodiment, even when no examination report is produced upon completion of the examination, the examination report is automatically produced and automatically transmitted to the workstation 50. In this way, the examination report is stored in the storage unit 60 of the workstation 50. Therefore, in a case where the user issues the instruction to start report production together with designation of a specific examination to the workstation 50, the workstation 50 retrieves the examination report for the designated specific examination stored in the storage unit 60 rather than producing a new examination report for that specific examination. The retrieved examination report is the object to be edited. The workstation 50 is therefore prevented from producing another new examination report for the designated specific examination. According to this embodiment, the ultrasonic diagnostic device 10 and the workstation 50 are prevented from independently producing different examination reports for the same examination.

Further, according to this embodiment, the storage location is identified based on the transmission/reception status of an examination report, and the examination report is acquired from the identified storage location. The examination report, which has been transmitted to the workstation 50, is acquired from the workstation 50 even when the storage unit 22 stores that examination report. In this way, centralized management of examination report can be implemented. Specifically, the examination report produced or edited in the ultrasonic diagnostic device 10 is automatically transmitted to the workstation 50. After the transmission, the ultrasonic diagnostic device 10 should acquire from the workstation 50 the transmitted examination report to edit the examination report in the ultrasonic diagnostic device 10. Thus, the examination report is centrally managed in the workstation 50. For example, in a case where, after the examination report for a particular examination is edited in the workstation 50, the instruction to start production of the examination report for the particular examination is issued to the ultrasonic diagnostic device 10, the ultrasonic diagnostic device 10 acquires the edited examination report from the workstation 50. This allows the ultrasonic diagnostic device 10 to edit the latest examination report for the particular examination.

Next, a modification example will be described. In the modification example, operation to edit the examination report in the workstation 50 is restricted based on the transmission/reception status of the examination report.

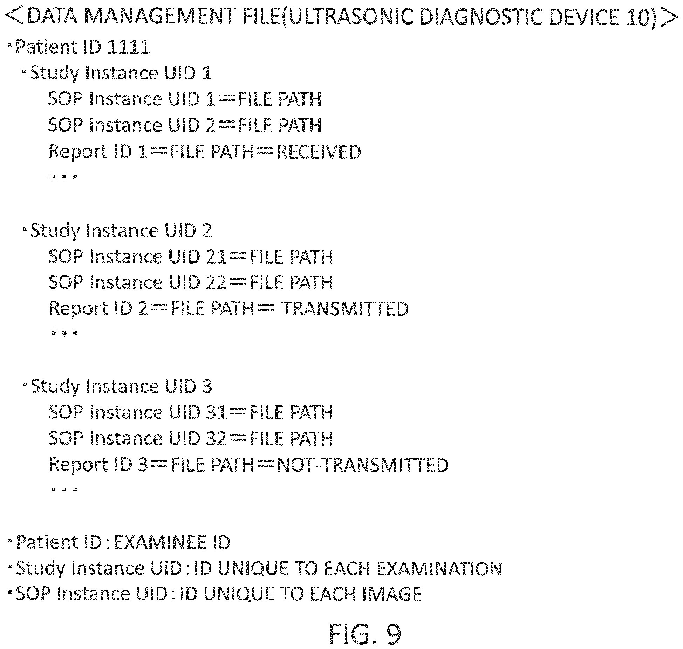

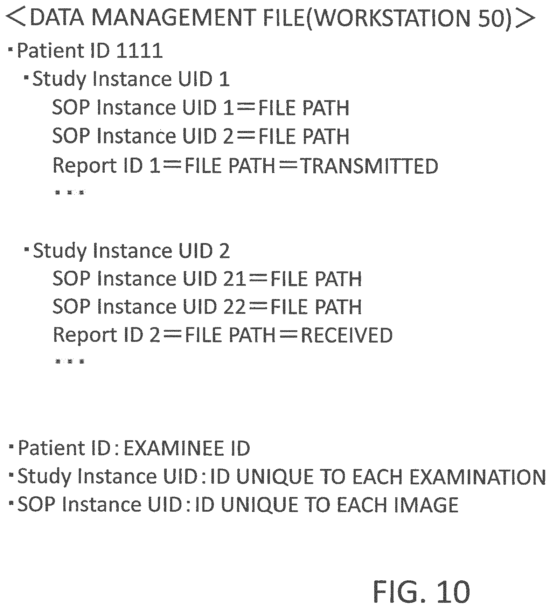

FIG. 9 shows an ultrasonic diagnostic device data management file. FIG. 10 shows a workstation data management file. The ultrasonic diagnostic device data management file is stored in the storage unit 22 of the ultrasonic diagnostic device 10. The transmission/reception manager unit 36 in the ultrasonic diagnostic device 10 generates the ultrasonic diagnostic device data management file. The workstation data management file is stored in the storage unit 60 of the workstation 50. The data manager unit 56 in the workstation 50 generates the workstation data management file.

The above-described data management files are configured in a manner similar to the data management file shown in FIG. 2. In the modification example, the data management files are used for managing the transmission/reception status of the examination report. Specifically, transmission/reception status information is associated with the report ID.

For example, the text "transmitted" in the data management file represents that a device owning the data management file has transmitted the examination report to another device and does not receive that examination report from the other device after the examination report has been transmitted. When the ultrasonic diagnostic device 10 transmits a particular examination report to the workstation 50, for example, the transmission/reception manager unit 36 defines the transmission/reception status of the particular examination report as "transmitted" in the ultrasonic diagnostic device data management file. In the example shown in FIG. 9, the transmission/reception status of the examination report having a report ID "2" is defined as "transmitted". This indicates that the examination report having the report ID "2" has been transmitted from the ultrasonic diagnostic device 10 to the workstation 50. Further, when the workstation 50 transmits a particular examination report to the ultrasonic diagnostic device 10, the data manager unit 50 defines the transmission/reception status of the particular examination report as "transmitted" in the workstation data management file. In the example shown in FIG. 10, the transmission/reception status of the examination report having a report ID "1" is defined as "transmitted". This means that the examination report having the report ID "1" is transmitted from the workstation 50 to the ultrasonic diagnostic device 10.

The text "receipt" in the data management file represents that the device owning the data management file has acquired the examination report from another device and does not transmit that examination report to the other device after the examination report is acquired. For example, when the ultrasonic diagnostic device 10 acquires a particular examination report from the workstation 50, the transmission/reception manager unit 36 defines, in the ultrasonic diagnostic device data management file, the transmission/reception status of the acquired particular examination report as "received". In the example shown in FIG. 9, the transmission/reception status of the examination report having the report ID "1" is defined as "receipt". This indicates that the ultrasonic diagnostic device 10 has acquired the examination report having the report ID "1" from the workstation 50. Then, when the workstation 50 acquires the examination report from the ultrasonic diagnostic device 10, i.e. the examination report is transmitted from the ultrasonic diagnostic device 10 to the workstation 50 as described in the above embodiment, the data manager unit 56 defines the transmission/reception status of the acquired examination report as "receipt" in the workstation data management file. In the example shown in FIG. 10, the examination report having the report ID "2" is defined as "receipt". This indicates that the workstation 50 has acquired the examination report having the report file "2" from the ultrasonic diagnostic device 10. In other words, the examination report is transmitted from the ultrasonic diagnostic device 10 to the workstation 50.

The text "not-transmitted" indicates that the ultrasonic diagnostic device 10 does not transmit the examination report to the workstation 50. In the example shown in FIG. 9, the transmission/reception status of the examination report having a report ID "3" is defined as "not-transmitted". This means that the examination report having the report ID "3" is not transmitted to the ultrasonic diagnostic device 10 to the workstation 50. As in the case of the above embodiment, upon completion of an examination, the examination report for the examination is transmitted from the ultrasonic diagnostic device 10 to the workstation 50. In this case, the transmission/reception manager unit 36 changes the transmission/reception status of the transmitted examination report from "not-transmitted" to "transmitted".

The transmission/reception status in the ultrasonic diagnostic device 10 and that in the workstation 50 correspond to each other. In particular, the status of "transmitted" corresponds to the status of "receipt". When the transmission/reception status in the ultrasonic diagnostic device 10 is defined as "transmitted", the transmission/reception status in the workstation 50 should be defined as "receipt". When the transmission/reception status in the ultrasonic diagnostic device 10 is defined as "transmitted", the transmission/reception status in the workstation 50 should be defined as "receipt". As an example, the examination report having the report ID "1" is explained in detail below. The transmission/reception status of the examination report having the report ID "1" in the ultrasonic diagnostic device 10 is defined as "received" (see FIG. 9), while the transmission/reception status of the examination report in the workstation 50 is defined as "transmitted" (see FIG. 10). This indicates that the examination report is transmitted from the workstation 50 to the ultrasonic diagnostic device 10, and received in the ultrasonic diagnostic device 10. Another example is explained with reference to the examination report having the report ID "2". The transmission/reception status of the examination report having the report ID "2" is defined as "transmitted" in the ultrasonic diagnostic device 10 (see FIG. 9), while that of the examination report having the report ID "2" is defined as "receipt" in the workstation 50 (see FIG. 10). This indicates that the examination report is transmitted from the ultrasonic diagnostic device 10 to the workstation, and received in the workstation 50.

In the modification example, when the transmission/reception status of a particular examination report is defined as "transmitted" in the workstation data management file, the controller unit 54 in the workstation 50 restricts the report production unit 58 from editing the particular examination report. This disables editing operation of the workstation 50. For example, the user is prohibited from editing the observations (comments), adding an ultrasound image, and performing other processing.

When the transmission/reception status of the examination report is defined as "transmitted" in the workstation data management file, the examination report has been transmitted from the workstation 50 to the ultrasonic diagnostic device 10. In this case, it is expected that the examination report is to be edited in the ultrasonic diagnostic device 10. That is, as in the case of the above-described embodiment, in a case where it is intended to edit the examination report in the ultrasonic diagnostic device 10, and the intended examination report has been transmitted to the workstation 50, the ultrasonic diagnostic device 10 acquires the intended examination report from the workstation 50. In such a case, the editing operation is disabled in the workstation 50, which can prevent the ultrasonic diagnostic device 10 and the workstation 50 from simultaneously performing edit operation. In this way, centralized management of the examination report can be implemented. Specifically, the transmission/reception status defined in the workstation data management file indicates whether or not the workstation 50 has authority to edit the examination report. When the transmission/reception status is defined as "transmitted", the workstation 50 has no edit authority, whereas when the transmission/reception status is defined as "receipt", the workstation 50 has edit authority. Thus, centralized management of the examination report can be implemented by restricting the workstation 50 from having edit authority.

After edit operation is finished in the ultrasonic diagnostic device 10, the edited examination report is transmitted from the ultrasonic diagnostic device 10 to the workstation 50. In this case, the transmission/reception status of the edited and transmitted examination report is defined as "transmitted" in the ultrasonic diagnostic device data management file. On the other hand, the transmission/reception status of the edited and transmitted examination report is defined as "receipt" in the workstation data management file. Under this condition, edit operation is enabled in the workstation. Meanwhile, in a case where edit operation is performed in the ultrasonic diagnostic device 10, the examination report to be edited is transmitted from the workstation 50 to the ultrasonic diagnostic device 10. Then, in the workstation data management file, the transmission/reception status of the transmitted examination report is defined as "transmitted", and edit operation is disabled in the workstation 50.

### [Another Embodiment]

In the above-described embodiment and modification example, when the examination report is displayed on the display unit 20, one or more ultrasound images to be displayed may be selected. For example, the report production unit 32 in the ultrasonic diagnostic device 10 executes a first display function and a second display function which will be described below. When the first display function is executed, among all the ultrasound images attached to the examination report, only those stored in the ultrasonic diagnostic device 10 are displayed in the examination report. When the second display function is executed, all the ultrasound images are displayed in the examination report. The report production unit 32 executes one of the first and second display functions selected by the user.

As in the case with the above-described embodiment, when the image UID is described in the examination report to be displayed, the report production unit 32 in the ultrasonic diagnostic device 10 refers to the data management file stored in the storage unit 22, to identify the file path associated with the described image UID.

When the first display function is selected, the report production unit 32 acquires data of only ultrasound images to which the ultrasonic diagnostic device 10 is specified as the file path. In other words, the report production unit 32 acquires data of only ultrasound images stored in the ultrasonic diagnostic device 10. For example, the report production unit 32 acquires data of the ultrasound images from the storage unit 22 in the ultrasonic diagnostic device 10. The report production unit 32 embeds data of the acquired ultrasound images in a predetermined position of the examination report, and displays the examination report embedded with the ultrasound images on the display unit 20. Even through there is data of an ultrasound image for which an external device, such as the workstation 50 is specified as the file path, the report production unit 32 does not acquire data of that ultrasound image from the external device, such as the workstation 50. In the examination report, its display region allocated to that ultrasound image is displayed as a blank region. It should be noted that the image UID of the not-displayed ultrasound image is retained in the examination report unless the image UID is deleted by the user.

When the second display function is selected, the report production unit 32 acquires data of all ultrasound images attached to the examination report. When the storage unit 22 of the ultrasonic diagnostic device 10 is specified as the file path for data of the ultrasound images, the report production unit 32 acquires data of the ultrasound images from the storage unit 22. When the workstation 50 is specified as the file path for data of the ultrasound images, the report production unit 32 acquires via the communication unit 24 data of the ultrasound images from the workstation 50. The report production unit 32 embeds data of the acquired ultrasound images in the predetermined position of the examination report, and displays the examination report embedded with data of the ultrasound images on the display unit 20. It should be noted when data of all the ultrasound images is transmitted from the ultrasonic diagnostic device 10 to the workstation 50, the report production unit 32 may acquire data of all the ultrasound images from the workstation 50. In this way, even when data of the ultrasound images is deleted in the ultrasonic diagnostic device 10, the deleted ultrasound images can be displayed on the examination report.

### [Further Embodiment]

In the above-described embodiment and modification example, in operation to add one or more ultrasound images in the examination report, the ultrasound images to be displayed may be selected. For example, the report production unit 32 in the ultrasonic diagnostic device 10 executes a third display function or a fourth display function which are described below. The report production unit 32 executes one of the third and fourth display functions selected by the user.

When the third display function is selected, the report production unit 32 displays, among all the ultrasound images associated with a specific examination designated by the user, only a group of ultrasound images stored in the ultrasonic diagnostic device 10 on the display unit 20. Using the input unit 40, the user can select one or more ultrasound images to be added to the examination report from the displayed group of ultrasound images.

When the fourth display function is selected, the report production unit 32 displays all ultrasound images associated with the specific examination on the display unit 20. For example, the report production unit 32 acquires data of the all ultrasound images associated with the specific examination via the communication unit 24 from the workstation 50. The report production unit 32 displays all the ultrasound images on the display unit 20. Using the input unit 40, the user can select one or more ultrasound images to be added to the examination report from all the displayed ultrasound images. Even when data of the ultrasound images is deleted in the ultrasonic diagnostic device 10, the ultrasound images can be added to the examination report as long as data of the ultrasound images is stored in the workstation 50.

Components other than the probe 12 in the above-described ultrasonic diagnostic device 10 may be implemented by means of hardware resources, such as, for example, a processor and an electronic circuit. In the implementation, a device, such as a memory, may be used as needed. Further, the components other than the probe 12 may be implemented, for example, by means of a computer. That is, the hardware resources, such as a CPU, a memory, and a hard disc, installed in the computer, may cooperate with software (programs) specifying operation of the CPU, and others, to thereby implement all or some of the components other than the probe 12. The programs are stored in a not-illustrated storage device via a storage medium, such as a CD or a DVD, or via a communication channel such as a network. As another example, the components other than the probe 12 may be implemented by a DSP (Digital Signal Processor), an FPGA (Field Programmable Gate Array), or the like.

The above-described workstation 50 may be implemented by means of hardware resources, such as, for example, a processor and an electronic circuit. In the implementation, a device, such as a memory, may be used as needed. Further, the workstation 50 may be implemented, for example, by a computer. That is, the hardware resources, such as a CPU, a memory, and a hard disc, installed in the computer, may cooperate with software (programs) specifying operation of the CPU and others, to thereby implement the workstation 50 entirely or partially. The programs are stored in a not-illustrated storage device via a storage medium, such as a CD or a DVD, or via a communication channel such as a network. As another example, the workstation 50 may be implemented by a DSP (Digital Signal Processor), an FPGA (Field Programmable Gate Array), or the like.

### REFERENCE SIGNS LIST

10 ultrasonic diagnostic device; 12 probe; 14 transmitter/receiver unit; 16 signal processor unit; 18 image generation unit; 20, 64 display unit; 22, 60 storage unit; 24, 52 communication unit; 26, 54 controller unit; 28 image manager unit; 30 measurement unit; 32, 58 report production unit; 34 report manager unit; 36 transmission/reception manager unit; 38 report acquisition unit; 40, 62 input unit; 50 workstation; 56 data manager unit.

## Claims

1. An ultrasonic diagnostic device, comprising:
a report production unit that produces an examination report under a condition that duplicate production is restricted to prevent presence of multiple examination reports for one examination;
a first manager unit that activates the report production unit upon receipt of an instruction to start report production from a user, and
a second manager unit that causes the report production unit to produce the examination report when no examination report is produced upon completion of an examination.

2. The ultrasonic diagnostic device according to Claim 1, further comprising:
a transmission unit that transmits, when the examination report is produced, the produced examination report to an external device equipped with another report production unit;
a transmission manager unit that manages each examination report in terms of whether or not the examination report is transmitted, and
a report acquisition unit that acquires, when an instruction to restart examination report production is received together with designation of a specific examination, the examination report associated with the specific examination, wherein the examination report associated with the specific examination is acquired from the external device when that examination report has been transmitted, or retrieved within the ultrasonic diagnostic device when that examination report is not transmitted.

3. The ultrasonic diagnostic device according to Claim 2, wherein:
when the examination report associated with the specific examination is not transmitted again to the external device after the report acquisition unit has acquired the examination report associated with the specific examination, the external device is restricted from editing the examination report associated with the specific examination.

4. The ultrasonic diagnostic device according to Claim 1, wherein:
the report production unit has a function of reflecting measurement data obtained in a present examination in the examination report.

5. The ultrasonic diagnostic device according to Claim 2, wherein:
in operation to transmit the examination report, the transmission unit transmits the examination report together with image data obtained in an examination associated with the examination report to be transmitted.

6. An examination report producing method, comprising:
a report production step of producing an examination report with a report producing function under a condition that duplicate production is restricted to prevent presence of multiple examination reports for one examination;
a first management step of activating the report producing function upon receipt of an instruction to start report production from a user, and
a second management step of causing the report producing function to produce the examination report when no examination report is created upon completion of an examination.
